# FASCICULE DE BREVET EUROPEEN

(11) **EP 0 348 275 B1**
(45) Date de publication et mention de la délivrance du brevet: **16.02.1994**
(21) Numéro de dépôt: 89401674.0
(22) Date de dépôt: 15.06.1989
(51) Int. Cl.: C12M 1/00

(54) **Procédé et installation pour l'obtention de plasmides et de cosmides**
Verfahren und Anlage zur Gewinnung von Plasmiden und Cosmiden
Method and apparatus for obtaining plasmids and cosmids

(30) Priorité: 21.06.1988 FR 8808306
(43) Date de publication de la demande: 27.12.1989
(73) Titulaire: BERTIN & CIE, F-78373 Plaisir Cédex (FR)
(72) Inventeur: Cohen, Daniel, F-94160 St Mande (FR); Dufau Frédéric, F-78170 La Celle St Cloud (FR); Hache, Jean, F-78960 Voisins le Bretonneux (FR)
(74) Mandataire: Orès, Bernard

(56) Documents cités:
- MOLECULAR CLONING, "A laboratory manual", éditio par T.E.F. Maniatis et al.,1982, pages 365-370, Cold Spring Harbor Laboratory, Cold Spring Harbor, NewYork, US

## Description

La présente invention est relative à un procédé et à une installation pour l'obtention de plasmides et cosmides entiers.

Les plasmides sont des molécules d'ADN double brin, circulaires, extrachromosomales, que l'on trouve dans beaucoup de souches bactériennes. Leur taille varie de 1 kbase à 200-300 kbases et est beaucoup plus petite que celle des chromosomes, par exemple.

Dans l'Art antérieur, il est connu de séparer les plasmides présents dans diverses bactéries, par centrifugation. La centrifugation des cellules bactériennes, ne permet d'obtenir qu'un rendement faible en plasmides, de l'ordre de 20 %. Or, les plasmides présentent, à l'heure actuelle, de nombreuses applications, notamment comme sondes d'hybridation ou comme vecteurs. Le besoin en plasmides devenant de plus en plus important, la possibilité d'obtenir des plasmides entiers avec un rendement très élevé, présente donc un intérêt pratique certain pour les industries concernées.

La Demanderesse s'est, en conséquence, donné pour but de pourvoir à un procédé spécifique d'obtention de plasmides et cosmides entiers, qui ne présente pas les inconvénients des procédés proposés dans l'Art antérieur, notamment en ce qu'il permet d'obtenir un rendement proche de 100 % en plasmides et cosmides entiers, et en ce qu'il est facile à mettre en oeuvre et peu coûteux, et à une installation automatisée pour la mise en oeuvre de ce procédé.

La présente invention a pour objet un procédé d'obtention de plasmides et de cosmides entiers présents dans des cellules bactériennes, cultivées dans un milieu approprié, caractérisé en ce qu'au cours d'une première étape lesdites cellules sont séparées de leur milieu de culture, en ce qu'au cours d'une deuxième étape, lesdites cellules sont incluses dans un gel approprié, leurs parois étant alors lysées par mise en contact avec un réactif approprié, puis lesdites cellules incluses sont solubilisées à l'aide d'au moins un réactif approprié, et en ce qu'au cours d'une troisième étape, lesdites cellules solubilisées, incluses dans ledit gel, sont soumises à un champ électrique approprié, provoquant leur migration électrophorétique, laquelle isole spécifiquement les plasmides et cosmides entiers présents dans lesdites cellules.

La lyse des parois cellulaires est réalisée de manière connue et classique à l'aide de lysozyme.

Selon un mode de mise en oeuvre du procédé, la lyse des parois cellulaires est réalisée avant la solidification du gel d'inclusion.

Selon un autre mode de mise en oeuvre du procédé, la lyse des parois cellulaires est réalisée après solidification du gel d'inclusion.

Selon un autre mode de mise en oeuvre, le gel d'inclusion est de l'agarose à une concentration comprise entre 0,1 % et 5%, sans que ces indications aient, bien entendu, quelque caractère limitatif que ce soit.

Selon un autre mode de mise en oeuvre du procédé, la solubilisation est réalisée chimiquement ou biochimiquement à l'aide d'un réactif choisi dans le groupe qui comprend des enzymes protéolytiques appropriées et des détergents solvants des lipides.

On peut citer notamment comme enzyme, la protéinase K, la pronase, ou toute autre protéase appropriée ; ou peut citer comme détergent approprié, notamment le dodécyl sulfate de sodium (SDS) ; l'association protéinase K-SDS, par exemple, est très avantageuse.

Selon une disposition avantageuse de ce mode de mise en oeuvre, ladite solubilisation est réalisée avant solidification du gel.

Selon une autre disposition avantageuse de ce mode de mise en oeuvre, ladite solubilisation est réalisée après solidification du gel.

Selon un autre mode de mise en oeuvre du procédé, la durée de migration électrophorétique est comprise entre deux et douze heures.

Selon une disposition avantageuse de ce mode de mise en oeuvre, la migration électrophorétique est réalisée de manière connue en champs pulsés.

Dans un tel mode d'exécution, on prévoit, bien entendu, le refroidissement du système de migration par des moyens de réglage thermique, lesquels servent avantageusement aussi, en cas de besoin, lors de la solubilisation des cellules, lorsque ceci est nécessaire.

La présente invention a également pour objet une installation pour l'obtention de plasmides et cosmides entiers présents dans des cellules bactériennes, caractérisée en ce qu'elle comprend au moins un moyen de séparation des cellules de leur milieu de culture, au moins une enceinte de traitement, au moins un gel d'électrophorèse servant de milieu de migration électrophorétique, adjacent et associé à ladite enceinte de traitement, au moins un moyen d'application d'un champ électrique provoquant la migration électrophorétique, des moyens amovibles et/ou éclipsables de mise en communication dudit gel d'électrophorèse avec ladite enceinte de traitement et un microprocesseur d'automatisation pour la commande séquentielle dudit moyen de séparation, desdits moyens de mise en communication et dudit moyen d'application du champ électrique.

Selon un mode de réalisation avantageux, l'installation comprend en outre au moins une enceinte de culture desdites cellules et un moyen de transfert desdites cellules de l'enceinte de culture dans l'enceinte de traitement, ledit moyen de transfert comportant des tubes de transfert reliés d'une part, à l'enceinte de culture, et d'autre part, à l'enceinte de traitement et un dispositif à pression différentielle entre ladite enceinte de culture et ladite enceinte de traitement.

Selon une disposition de ce mode de réalisation, ledit dispositif est une pompe péristaltique.

Selon une autre disposition de ce mode de réalisation, ledit dispositif est un surpresseur relié à l'enceinte de culture.

Selon encore une autre disposition de ce mode de réalisation, ledit dispositif est une pompe à vide reliée à l'enceinte de traitement.

Selon un autre mode de réalisation de l'installation, l'enceinte de traitement est avantageusement un bac, dont le fond comporte le moyen de séparation constitué par une membrane de porosité appropriée à la séparation de molécules de poids moléculaire supérieur à 80 kDa.

Dans un tel mode de réalisation, le bac comprend trois parois fixes dont l'une, adjacente à une des électrodes du moyen d'application d'un champ électrique, est une membrane de dialyse et les moyens amovibles de mise en communication du gel d'électrophorèse avec le bac de traitement sont alors, le plus simplement, une réglette matérialisant la quatrième paroi dudit bac, mise en place pour la coulée du gel dans le bac de traitement.

Dans une variante, le bac est limité par quatre réglettes amovibles simultanément, mises en place pour la coulée du gel et une des électrodes du moyen d'application d'un champ électrique est alors, éventuellement, adjacente à une membrane de dialyse.

Dans une réalisation préférée, la pompe à vide assurant le transfert des cellules de l'enceinte de culture audit bac de traitement fournit simultanément la dépression d'aspiration du milieu de culture au travers de ladite membrane.

L'invention comprend avantageusement un détecteur localisant l'emplacement des plasmides et cosmides entiers après ladite migration électrophorétique.

On obtient ainsi, par la combinaison de moyens de la présente invention, une séparation automatisée rapide et facile à mettre en oeuvre de plasmides et de cosmides entiers.

Outre les dispositions qui précèdent, l'invention comprend encore d'autres dispositions, qui ressortiront de la description qui va suivre, qui se réfère à des exemples de mise en oeuvre du procédé conforme à l'invention et à une description détaillée de l'installation avec référence au dessin annexé, dans lequel :
- la figure 1 représente une vue schématique illustrant le procédé de l'invention ;
- la figure 2 représente une vue très schématique, en coupe longitudinale, illustrant un mode de réalisation d'une partie de l'installation ;
- la figure 3 représente une vue très schématique, en coupe longitudinale, illustrant un autre mode de réalisation d'une partie de l'installation.

Il doit être bien entendu, toutefois, que ce dessin et les parties descriptives correspondantes, ainsi que les exemples, sont donnés uniquement à titre d'illustration de l'objet de l'invention, dont ils ne constituent en aucune manière une limitation.

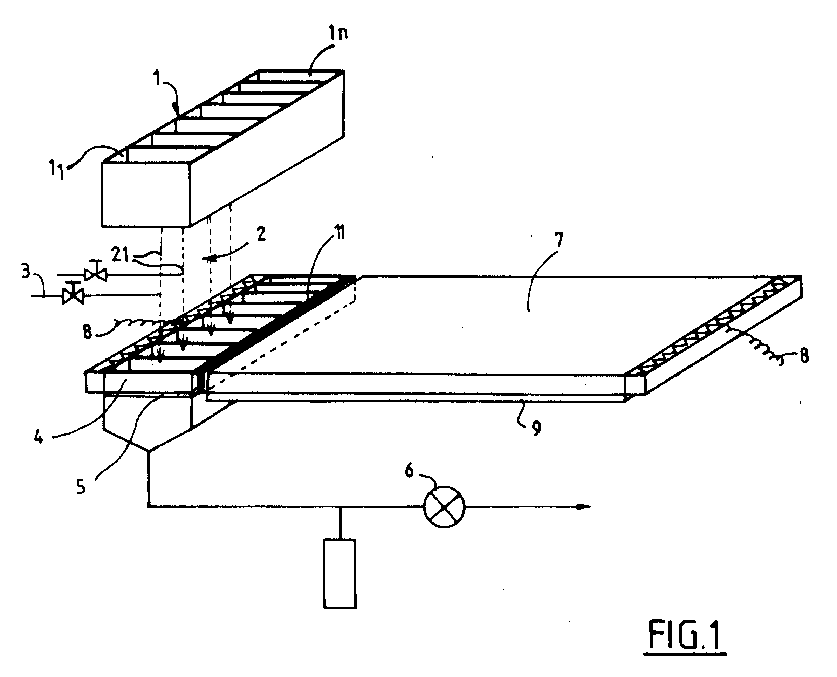

Le procédé selon l'invention, d'obtention de plasmides et de cosmides est illustré sur la figure 1. Des bactéries hôtes de plasmides ou de cosmides sont sélectionnées et cultivées de manière appropriée, dans une enceinte de culture cellulaire 1 comportant un certain nombre de compartiments 1₁ à 1ₙ permettant la croissance en parallèle de cellules bactériennes différentes hébergeant des plasmides ou cosmides différents. Après avoir obtenu une concentration cellulaire convenable, lesdites cultures sont transférées dans le bac de traitement 4 par l'intermédiaire des moyens de transfert 2, qui dans la réalisation représentée sont constitués par des tubes 21 et une pompe à vide 6, le bac de traitement 4 étant alors muni d'un couvercle non représenté. La détermination de la concentration cellulaire peut être réalisée de diverses manières et notamment, mais non limitativement, par une mesure colorimétrique. Les cellules sont ensuite séparées de leur milieu de culture, le moyen de séparation étant représenté par le fond du bac de traitement 4 qui est une membrane appropriée 5, par exemple, et de manière non limitative, une membrane d'ultrafiltration en "Teflon" (une marque déposée de la Société Du Pont de Nemours) qui retient notamment les éléments de plus de 80 kDa.

La pompe à vide 6 assure à la fois le transfert des cellules de l'enceinte de culture au bac de traitement et fournit également la dépression d'aspiration et d'évacuation du milieu de culture au travers de ladite membrane. Une série de pipettes 3 ₁ à 3 ₙ reliée aux tubes 21 permet l'introduction des réactifs et notamment de l'agarose, qui est alors introduit à chaud, à une température d'environ 65°C et permet l'inclusion desdites cellules ; les parois cellulaires sont ensuite lysées, par exemple à l'aide de lysozyme ; ce dernier est maintenu en contact avec lesdites cellules pendant 5 à 10 minutes. Cette lyse peut être réalisée indifféremment avant solidification du gel d'inclusion, à une température de l'ordre de 37°C ou après solidification du gel d'inclusion.

Les cellules sont alors solubilisées, la solubilisation pouvant être réalisée indifféremment avant ou après solidification du gel d'inclusion, à l'aide d'une enzyme appropriée, éventuellement associée à un détergent, par exemple, mais non limitativement de la protéinase K et du SDS.

Après la solubilisation, la migration électrophorétique, en champs pulsés ou non, est réalisée ; le bac de traitement 4 est alors relié à la plaque de gel d'électrophorèse 7 par le retrait de la paroi mobile 11 qui dans la réalisation représentée est une des parois amovibles du bac de traitement 4, et l'espace entre le gel contenant les cellules traitées et le gel d'électrophorèse est rempli par du gel d'électrophorèse. La paroi 12 du bac de traitement 4 est une membrane de dialyse. Des électrodes 8 permettent l'obtention d'un champ électrique approprié à la migration électrophorétique recherchée.

Le bac de traitement 4 et la plaque de gel d'électrophorèse 7 sont placés dans un récipient 30, dans lequel se trouve le tampon gel d'électrophorèse et les électrodes 8. Un détecteur 9 permet de localiser l'emplacement de migration des plasmides et des cosmides.

Le procédé conforme à l'invention permet dans un temps court, compris entre deux et douze heures, d'obtenir spécifiquement les plasmides et les cosmides, ceux-ci migrant sur le gel alors que, d'une part, les chromosomes restent au niveau du dépôt et que, d'autre part, les produits de la solubilisation (peptides, ...) ne sont pas retenus par le gel.

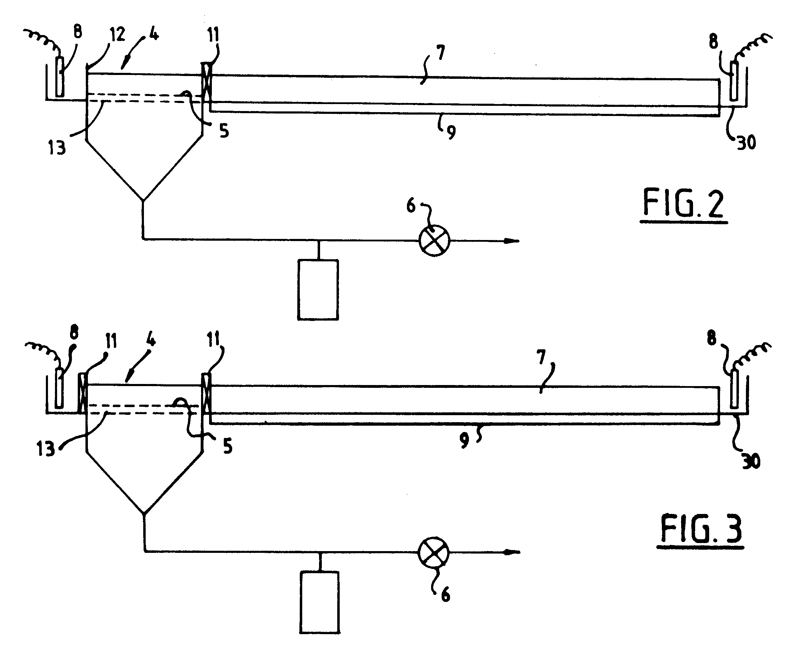

La figure 2 représente une vue très schématique, en coupe longitudinale, d'un mode de réalisation d'une partie de l'installation et comporte un bac de traitement 4 comprenant une paroi amovible 11 et trois parois fixes, dont celle, qui est adjacente à l'électrode 8 se trouvant près du bac de traitement 4, est une membrane de dialyse 12.

Le réceptacle 30 du bac de traitement 4 et du gel d'électrophorèse 7 comprend :
- un support approprié 13, sur lequel se trouve le fond du bac de traitement 4, sous forme d'une membrane appropriée 5, par exemple et de manière non limitative, une membrane d'ultrafiltration en "Teflon" qui retient notamment les éléments de plus de 80 kDa ;
- le moyen d'application du champ électrique, représenté par les électrodes 8.

Au moyen de séparation 5 des cellules de leur milieu de culture, est associée la pompe à vide 6.

Un détecteur 9 permet de localiser l'emplacement de migration des plasmides et des cosmides.

La figure 3 représente une vue très schématique, en coupe longitudinale, d'un autre mode de réalisation d'une partie de l'installation et comporte un bac de traitement 4 comprenant deux parois amovibles 11 et deux parois fixes.

Le réceptacle 30 du bac de traitement 4 et du gel d'électrophorèse 7 comprend :
- un support approprié 13, sur lequel se trouve le fond du bac de traitement 4, sous forme d'une membrane appropriée 5, par exemple et de manière non limitative, une membrane d'ultrafiltration en "Teflon" qui retient notamment les éléments de plus de 80 kDa ;
- le moyen d'application du champ électrique, représenté par les électrodes 8.

Au moyen de séparation 5 des cellules de leur milieu de culture, est associée la pompe à vide 6.

Un détecteur 9 permet de localiser l'emplacement de migration des plasmides et des cosmides.

De telles installations permettent la mise en oeuvre du procédé d'obtention de plasmides et de cosmides conforme à l'invention.

### Exemple 1 :

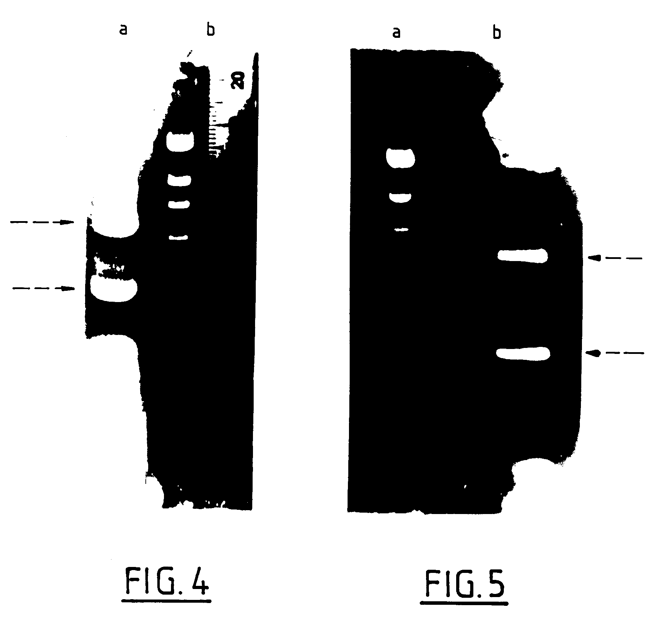

On cultive des bactéries contenant le plasmide à isoler, dans un milieu LB (bactotryptone, bacto yeast extract et NaCl) contenant un antibiotique, à 37°C dans l'enceinte de culture 1, puis on transfère lesdites bactéries dans le bac de traitement 4 et on élimine le milieu de culture en filtrant par passage sur la membrane 5, en faisant agir la pompe à vide 6. On coule de l'agarose à 1 %, maintenu à 37°C dans le bac de traitement 4 ; on laisse refroidir 15 minutes, afin que l'agarose contenant les bactéries polymérise et on incube le bloc obtenu 10 minutes à température ambiante dans une solution de lysozyme à 6 mg/ml. On ajoute une solution de protéinase K à 5 mg/ml et du sodium dodécyl sulfate à 10 % et par mise en oeuvre des moyens de réglage thermique, on incube 1 heure à 42°C. Les cellules, ainsi traitées, sont prêtes à la migration électrophorétique qui est effectuée pendant 3 heures à 70 volts (environ 60 mA) ; on obtient alors, les plasmides désirés, comme représenté sur la Figure 4a (flèches) ; la colonne 4b représente un marqueur de taille (phage lambda digéré par HindIII).

### Exemple 2 :

On procède comme dans l'Exemple 1, la migration étant effectuée pendant douze heures à 10 volts (environ 9 mA) ; on obtient alors les plasmides désirés comme représentées sur la Figure 5b (flèches) ; la colonne 5a représente un marqueur de taille (phage lambda digéré par HindIII).

Ainsi que cela ressort de ce qui précède, l'invention ne se limite nullement à ceux de ses modes de mise en oeuvre, de réalisation et d'applications qui viennent d'être décrits de façon plus explicite ; elle en embrasse, au contraire, toutes les variantes qui peuvent venir à l'esprit du technicien en la matière, sans s'écarter du cadre, ni de la portée de la présente invention.

## Revendications

1. Procédé d'obtention de plasmides et de cosmides entiers présents dans des cellules bactériennes, cultivées dans un milieu approprié, caractérisé en ce qu'au cours d'une première étape lesdites cellules sont séparées de leur milieu de culture, en ce qu'au cours d'une deuxième étape, lesdites cellules sont incluses dans un gel approprié, leurs parois étant alors lysées par mise en contact avec un réactif approprié, puis lesdites cellules incluses sont solubilisées à l'aide d'au moins un réactif approprié, et en ce qu'au cours d'une troisième étape, lesdites cellules solubilisées, incluses dans ledit gel, sont soumises à un champ électrique approprié provoquant leur migration électrophorétique, laquelle isole spécifiquement les plasmides et cosmides entiers présents dans lesdites cellules.

2. Procédé selon la revendication 1, caractérisé en ce que la lyse des parois cellulaires est réalisée avant la solidification du gel d'inclusion.

3. Procédé selon la revendication 1, caractérisé en ce que la lyse des parois cellulaires est réalisée après solidification du gel d'inclusion.

4. Procédé selon l'une quelconque des revendications 1 à 3, caractérisé en ce que le gel d'inclusion est de l'agarose à une concentration comprise entre 0,1 % et 5%.

5. Procédé selon l'une quelconque des revendications 1 à 4, caractérisé en ce que la solubilisation est réalisée chimiquement ou biochimiquement à l'aide d'un réactif choisi dans le groupe qui comprend des enzymes protéolytiques appropriées et des détergents solvants des lipides.

6. Procédé selon la revendication 5, caractérisé en ce que ladite solubilisation est réalisée avant solidification du gel.

7. Procédé selon la revendication 5, caractérisé en ce que ladite solubilisation est réalisée après solidification du gel.

8. Procédé selon l'une quelconque des revendications 1 à 7, caractérisé en ce que la durée de migration électrophorétique est de deux à six heures.

9. Procédé selon la revendication 8, caractérisé en ce que la migration électrophorétique est réalisée de manière connue en champs pulsés.

10. Installation pour l'obtention de plasmides et cosmides entiers présents dans des cellules bactériennes, caractérisée en ce qu'elle comprend au moins un moyen de séparation (5) des cellules de leur milieu de culture, au moins une enceinte de traitement (4), au moins un gel d'électrophorèse (7) servant de milieu de migration électrophorétique, adjacent et associé à ladite enceinte de traitement, au moins un moyen d'application d'un champ électrique (8) provoquant la migration électrophorétique, des moyens amovibles et/ou éclipsables (11) de mise en communication dudit gel d'électrophorèse avec ladite enceinte de traitement et un microprocesseur d'automatisation pour la commande séquentielle dudit moyen de séparation, desdits moyens de mise en communication et du moyen d'application du champ électrique.

11. Installation selon la revendication 10, caractérisée en ce qu'elle comprend en outre au moins une enceinte de culture (1) desdites cellules et un moyen de transfert (2) desdites cellules de l'enceinte de culture dans l'enceinte de traitement, ledit moyen de transfert comportant des tubes de transfert (21) reliés d'une part, à l'enceinte de culture, et d'autre part, à l'enceinte de traitement et un dispositif à pression différentielle entre l'enceinte de culture et l'enceinte de traitement.

12. Installation selon la revendication 11, caractérisée en ce que ledit dispositif est une pompe péristaltique.

13. Installation selon la revendication 11, caractérisée en ce que ledit dispositif est un surpresseur relié à l'enceinte de culture.

14. Installation selon la revendication 11, caractérisée en ce que, ledit dispositif est une pompe à vide reliée à l'enceinte de traitement.

15. Installation selon l'une quelconque des revendications 10 à 14, caractérisée en ce que l'enceinte de traitement est avantageusement un bac, dont le fond comporte le moyen de séparation (5) constitué par une membrane de porosité appropriée à la séparation de molécules de poids moléculaire supérieur à 80 kDa.

16. Installation selon la revendication 15, caractérisée en ce que le bac comprend trois parois fixes dont l'une, adjacente à une des électrodes (8) du moyen d'application d'un champ électrique, est une membrane de dialyse (12) et les moyens amovibles de mise en communication du gel d'électrophorèse avec le bac de traitement sont alors une réglette (11) matérialisant la quatrième paroi dudit bac, mise en place pour la coulée du gel dans le bac de traitement.

17. Installation selon la revendication 15, caractérisée en ce que le bac est limité par quatre réglettes (11) amovibles simultanément, mises en place pour la coulée du gel et en ce qu'une des électrodes (8) du moyen d'application d'un champ électrique est alors éventuellement adjacente à une membrane de dialyse (12).

18. Installation selon l'une quelconque des revendications 14 à 17, caractérisée en ce que la pompe à vide (6) assurant le transfert des cellules de l'enceinte de culture audit bac de traitement fournit simultanément la dépression d'aspiration du milieu de culture au travers de ladite membrane.

19. Installation selon l'une quelconque des revendications 10 à 18, caractérisée en ce qu'elle comprend un détecteur (9) localisant l'emplacement des plasmides et cosmides entiers après ladite migration électrophorétique.

20. Installation selon l'une quelconque des revendications 10 à 19, caractérisée en ce qu'elle comprend des moyens de réglage thermique de chauffage de l'enceinte de traitement (4) et/ou de refroidissement lors de la migration électrophorétique.

## Claims

1. Process for obtaining whole plasmids and cosmids present in bacterial cells, cultivated in a suitable medium, characterized in that in the course of a first step said cells are separated from their culture medium, in that in the course of a second step, said cells are included in a suitable gel, their walls then being lysed by contacting with a suitable reagent, then said included cells are solubilized by means of at least one suitable reagent, and in that in the course of a third step, said solubilized cells, included in said gel, are subjected to a suitable electrical field causing their electrophoretic migration, which specifically isolates the whole plasmids and cosmids present in said cells.

2. Process according to claim 1, characterized in that the lysis of the cell walls is performed before solidification of the inclusion gel.

3. Process according to claim 1, characterized in that the lysis of the cell walls is performed after solidification of inclusion gel.

4. Process according to any one of claims 1 to 3, characterized in that the inclusion gel is agarose at a concentration comprised between 0.1% and 5%.

5. Process according to any one of claims 1 to 4, characterized in that the solubilization is performed chemically or biochemically by means of a reagent selected from the group which comprises suitable proteolytic enzymes and solvent detergents of lipids.

6. Process according to claim 5, characterized in that said solubilization is performed before solidification of said gel.

7. Process according to claim 5, characterized in that said solubilization is performed after solidification of the gel.

8. Process according to any one of claims 1 to 7, characterized in that the duration of electrophoretic migration is from two to six hours.

9. Process according to claim 8, characterized in that the electrophoretic migration is performed in known manner in pulsed fields.

10. Installation for obtaining whole plasmids and cosmids present in bacterial cells, characterized in that it comprises at least one separating means (5) of the cells from their culture medium, at least one treatment chamber (4), at least one electrophoresis gel (7) serving as an electrophoretic migration medium, adjacent and associated with said treatment chamber, at least one means for applying an electric field (8) causing the electrophoretic migration, removable and/or retractable means (11) for placing in communication said electrophoresis gel with said treatment chamber and an automatization microprocessor for the sequential control of said separation means, of said means of placing in communication and of the means of applying the electric field.

11. Installation according to claim 10, characterized in that it comprises in addition at least one culture chamber (1) for said cells and a transfer means (2) for said cells from the culture chamber to the treatment chamber, said transfer means comprising transfer tubes (21) connected on the one hand, to the culture chamber, and on the other hand, to the treatment chamber and a differential pressure device between the culture chamber and the treatment chamber.

12. Installation according to claim 11, characterized in that said device is a peristaltic pump.

13. Installation according to claim 11, characterized in that said device is an excess pressure generator connected to the culture chamber.

14. Installation according to claim 11, characterized in that said device is a vacuum pump connected to the treatment chamber.

15. Installation according to any one of claims 10 to 14, characterized in that the treatment chamber is advantageously a tank, whose bottom comprises the separation means (5) constituted by a membrane of suitable porosity for the separation of molecules of molecular weight higher than 80 kDa.

16. Installation according to claim 15, characterized in that the tank comprises three fixed walls of which one, adjacent to one of the electrodes (8) of the means of applying the electric field, is a dialysis membrane (12) and the removable means for placing in communication the electrophoresis gel with the treatment tank are then a ruler (11) materialising the fourth wall of said tank, placed in position for the flow of the gel into the treatment tank.

17. Installation according to claim 15, characterized in that the tank is limited by four rules (11) simultaneously removable, placed in position for the flow of the gel and in that one of the electrodes (8) of the means of the application of an electric field is then possibly adjacent to a dialysis membrane (12).

18. Installation according to any one of claims 14 to 17, characterized in that the vacuum pump (6) ensuring the transfer of the cells from the culture chamber to said treatment tank supplies simultaneously the reduced pressure for aspiration from the culture medium through said membrane.

19. Installation according to any one of claims 10 to 18, characterized in that it comprises a detector (9) locating the site of the whole plasmids and cosmids after said electrophoretic migration.

20. Installation according to any one of claims 10 to 19, characterized in that it comprises means for thermal regulation of heating the treatment chamber (4) and/or for cooling during the electrophoretic migration.

## Patentansprüche

1. Verfahren zur Gewinnung von ganzen Plasmiden und Cosmiden, die in Bakterienzellen, gezüchtet in einem geeigneten Medium, vorhanden sind, dadurch gekennzeichnet, daß im Verlaufe eines ersten Schritte die Zellen von ihrem Nährmedium abgetrennt werden, daß in Verlaufe eines zweiten Schritte die Zeilen in ein geeignetes Gel eingeschlossen werden, wobei ihre wände dann durch Kontakt mit einem geeigneten Reagens lysiert werden, die eingeschlossenen Zellen daraufhin mit Hilfe mindestens eines geeigneten Reagens' solubilisiert werden, und daß im Verlaufe eines dritten Schritte die solubilisierten, im Gel eingeschlossenen Zellen einem geeigneten elektrischen Feld ausgesetzt werden, das ihre elektrophoretische Wanderung hervorruft, die spezifisch die in den Zellen vorhandenen, ganzen Plasmide und Cosmide isoliert.

2. Verfahren nach Anspruch 1, dadurch gekennzeichnet, daß die Lyse der Zellwände vor dem Erstarren des Einschließungsgels ausgeführt wird.

3. Verfahren nach Anspruch 1, dadurch gekennzeichnet, daß die Lyse der Zellwände nach dem Erstarren des Einschließungsgels ausgeführt wird.

4. Verfahren nach einem der Ansprüche 1 bis 3, dadurch gekennzeichnet, daß das Einschließungsgel aus Agarose mit einer Konzentration zwischen 0,1% und 5% besteht.

5. Verfahren nach einem der Ansprüche 1 bis 4, dadurch gekennzeichnet, daß die Solubilisierung chemisch oder biochemisch mit Hilfe eines Reagens' ausgeführt wird, das aus der Gruppe, bestehend aus geeigneten proteolytischen Enzymen und lipidlöslichen Detergentien, ausgewählt wird.

6. Verfahren nach Anspruch 5, dadurch gekennzeichnet, daß die Solubilisierung vor dem Erstarren des Gels ausgeführt wird.

7. Verfahren nach Anspruch 5, dadurch gekennzeichnet, daß die Solubilisierung nach dem Erstarren des Gels ausgeführt wird.

8. Verfahren nach einem der Ansprüche 1 bis 7, dadurch gekennzeichnet, daß die Dauer der elektrophoretischen Wanderung zwei bis sechs Stunden beträgt.

9. Verfahren nach Anspruch 8, dadurch gekennzeichnet, daß die elektrophoretische Wanderung in bekannter Weise mit Feldpulsen ausgeführt wird.

10. Anlage zur Gewinnung von in Bakterienzellen vorhandenen, ganzen Plasmiden und Cosmiden, dadurch gekennzeichnet, daß sie mindestens eine Einrichtung (5) zum Trennen der Zellen von ihrem Nährmedium, mindestens ein Verarbeitungsgehäuse (4), mindestens ein als Matrix für die elektrophoretische Wanderung dienendes Elektrophoresegel (7), das an das Verarbeitungsgehäuse angrenzt und mit ihm verbunden ist, mindestens eine Einrichtung (8) zum Anlegen eines elektrischen Feldes, das die elektrophoretische Wanderung hervorruft, eine verstellbare und/oder einziehbare Einrichtung (11) zum Herstellen einer Verbindung des Elektrophoresegels mit dem Verarbeitungsgehäuse und einen Automatisierungsmikroprozessor für die Ablaufsteuerung der Trenneinrichtung, der Einrichtung zur Herstellung einer Verbindung und der Einrichtung zum Anlegen eines elektrischen Feldes, umfaßt.

11. Anlage nach Anspruch 10, dadurch gekennzeichnet, daß sie ferner mindestens ein Zellkulturgefäß (1) und eine Einrichtung (2) zum Übertragen der Zellen vom Kulturgefäß in das Verarbeitungsgehäuse umfaßt, wobei die Übertragungseinrichtung Übertragungsröhrchen (21), die an einer Seite an dem Kulturgefäß und an der anderen Seite an dem Verarbeitungsgehäuse angeschlossen sind, und eine Differentialdruckvorrichtung zwischen Kulturgefäß und Verarbeitungsgehäuse aufweist.

12. Anlage nach Anspruch 11, dadurch gekennzeichnet, daß die Vorrichtung eine Peristaltikpumpe ist.

13. Anlage nach Anspruch 11, dadurch gekennzeichnet, daß die Vorrichtung ein an dem Kulturgefäß angeschlossener Kompressor ist.

14. Anlage nach Anspruch 11, dadurch gekennzeichnet, daß die Vorrichtung eine an dem Verarbeitungsgehäuse angeschlossene Vakuumpumpe ist.

15. Anlage nach einem der Ansprüche 10 bis 14, dadurch gekennzeichnet, daß das Verarbeitungsgehäuse vorteilhaft ein Kasten ist, dessen Boden die Trenneinrichtung (5) enthält, die aus einer porösen Membran, geeignet zum Trennen von Molekülen mit einem Molekulargewicht größer als 80 kDa, besteht.

16. Anlage nach Anspruch 15, dadurch gekennzeichnet, daß der Kasten drei feste Wände umfaßt, von denen eine, die an eine der Elektroden (8) der Einrichtung zum Anlegen eines elektrischen Feldes angrenzt, eine Dialysemembran (12) ist, und die verstellbare Einrichtung zur Herstellung einer Verbindung des Elektrophoresegels mit dem Verarbeitungskasten eine Leiste (11) ist, die die vierte, für das Hineinfließen des Gels in den Verarbeitungskasten aufgestellte Wand des Kastens verkörpert.

17. Anlage nach Anspruch 15, dadurch gekennzeichnet, daß der Kasten durch vier gleichzeitig verstellbaren Leisten (11) begrenzt ist, die für das Hineinfließen des Gels aufgestellt sind, und daß eine der Elektroden (8) der Einrichtung zum Anlegen eines elektrischen Feldes dann gegebenenfalls an eine Dialysemembran (12) angrenzt.

18. Anlage nach einem der Ansprüche 14 bis 17, dadurch gekennzeichnet, daß die Vakuumpumpe (6), die die Übertragung der Zellen vom Kulturgefäß zum Verarbeitungskasten sicherstellt, gleichzeitig den Ansaugunterdruck des Nährmediums quer über die Membran liefert.

19. Anlage nach einem der Ansprüche 10 bis 18, dadurch gekennzeichnet, daß sie einen Detektor (9) umfaßt, der die Lage der ganzen Plasmide und Cosmide nach der elektrophoretischen Wanderung lokalisiert.

20. Anlage nach einem der Ansprüche 10 bis 19, dadurch gekennzeichnet, daß sie eine Einrichtung zur thermischen Regulierung der Heizung des Verarbeitungsgehäuses (4) und/oder der Kühlung während der elektrophoretischen Wanderung umfaßt.
